# EUROPEAN PATENT SPECIFICATION

(11) **EP 0 353 710 B1**
(45) Date of publication and mention of the grant of the patent: **20.04.1994**
(21) Application number: 89114186.3
(22) Date of filing: 01.08.1989
(51) Int. Cl.: B01L 3/14, A61B 5/14, C08L 67/02

(54) **Blood collecting tube**
Blutprobenröhrchen
Tube collecteur de sang

(30) Priority: 03.08.1988 JP 193608/88
(43) Date of publication of application: 07.02.1990
(73) Proprietor: TERUMO KABUSHIKI KAISHA, Tokyo 151 (JP)
(72) Inventor: Kasai, Masaaki c/o Terumo Kabushiki Kaisha, Nakakoma-gun Yamanashi (JP); Yamazaki, Sakae c/o Terumo Kabushiki Kaisha, Nakakoma-gun Yamanashi (JP); Miyake, Sanae c/o Terumo Kabushiki Kaisha, Nakakoma-gun Yamanashi (JP)
(74) Representative: Casalonga, Axel

(56) References cited:
- EP-A- 0 059 297
- EP-A- 0 171 161
- EP-A- 0 193 279
- WO-A-88/04154

## Description

This invention relates to a blood collecting tube for collecting a blood sample for use in various blood tests.

Various blood collectors have been used in clinical laboratory tests such as biochemical examinations and serologic tests. Generally used blood collectors are of the type comprising a blood collecting tube whose interior is kept under reduced pressure, and a tube holder capable of receiving the blood collecting tube therein and provided with a puncture needle at the tip thereof. The blood collecting tube comprises a tubular member made of glass or synthetic resin which has an open end and a closed bottom, and a rubber plug for closing the open end of the tubular member.

A tubular member made of glass can maintain the reduced internal pressure thereof for a long time but it is easy to be damaged in transportation and operation. The damaged tubular member causes the contamination of the blood sample in the tubular member. Additionally, a glass tubular member is relatively heavy to handle. In contrast to this, a plastic tubular member made of synthetic resin is advantageous because it is light and hard to damage even when dropped.

A blood collecting tube using a conventional plastic tubular member has, however, the problem that the capability of collecting blood considerably decreases with time because of the insufficient gas barrier properties of the tubular member. Although there is known a plastic tubular member made of polyethylene terephthalate, it is also disadvantageous because polyethylene terephthalate is easy to whiten at the gate position upon injection moulding and stop up the gate of the injection moulding machine. It results in low productability.

Efforts have been made to find a raw material presenting sufficient gas-barrier properties for making tubular members of blood-collecting tubes. EP-A-O 193 279 discloses vacuum blood-collecting tubes where the tubular member is made of polyethylenterephthalate, a copolymer of polyethyleneterephthalate or an acrylonitrile resin and wrapped so as to be air-tight in a plastic wrapping acting as a gas-barrier. But such blood-collecting tubes showed nevertheless an insufficient capacity for collecting blood with time.

In the food industry, barrier properties of polyester containers to package liquids having a sensitivity to oxygen or liquids requiring internal pressurization by addition of carbon dioxide, needed to be improved. Researches have been made to find a material providing such properties and also being capable of being injection-moulded. EP-A-O 171 161 describes such a material consisting of a polyester-based resin blend comprising a polyethyleneterephthalate and a copolyester containing isophthalic acid, terephthalic acid, ethyleneglycol and 1,3-bis(2-hydroxyethoxy) benzene.

It has been surprisingly discovered that blood collecting tubes having their tubular member made of a synthetic resin comprising a polyethylene terephthalate and a copolyester formed of specific amounts of isophthalic acid, terephthalic acid, ethylene glycol and 1,3-bis(2-hydroxyethoxy) benzene or 1,4-bis(2-hydroxyethoxy) benzene components and which open end is provided with a particular closure member, showed only insignificant variations of their capacity for collecting blood with time, and their tubular member showed good gas barrier properties and could be easely injection-moulded.

Accordingly, it is an object of the present invention to provide a blood collecting tube comprising a tubular member which is made of synthetic resin having high gas-barrier properties so that the capability of collecting blood decreases little with time.

It is another object of the present invention to provide a blood collecting tube comprising a tubular member which is made of a synthetic resin suitable for injection moulding.

According to an aspect of the present invention, a blood collecting tube comprises a tubular member made of synthetic resin which has an open end and a closed bottom, and a closure member which is for closing the open end of the tubular member and allows a puncture needle to pierce therethrough, the interior of said blood collecting tube being to be kept under reduced pressure, said tubular member being essentially made of a mixture of a polyester resin mainly based on ethylene glycol and terephthalic acid and a polyester resin mainly based on ethylene glycol and isophthalic acid.

The above and other objects, features, and advantages of the present invention will be better understood from the following description taken in conjunction with the accompanying drawings, in which;
Fig.1 is a cross sectional view of a blood collecting tube according to one preferred embodiment of the present invention; and
Fig.2 is an enlarged fragmentary sectional view of a closure member of the blood collecting tube according to the present invention.

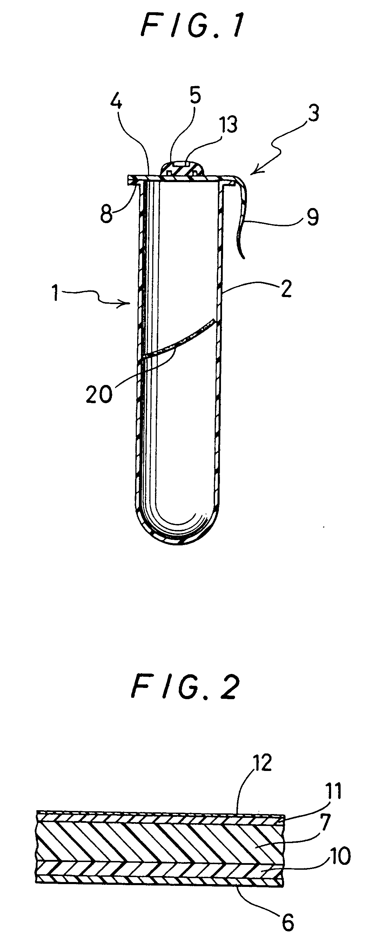

Referring first to Fig.1, a blood collecting tube 1 comprises a tubular member 2 which has an open end and a closed bottom, and a closure member 3 for closing the open end of the tubular member. The internal pressure of the blood collecting tube 1 is reduced in accordance with the amount of blood to be collected. The tubular member 2 is substantially cylindrical but the region of its closed bottom. An annular outward flange 8 is formed at the open end of the tubular member 2. The flange 8 projects out perpendicularly to the axis of the tubular member 2 in order to mount a gas-barrier member of the closure member 3 as will be described later.

The tubular member 2 is made of a polyester resin mixture having high gas barrier properties to keep the interior of the blood collecting tube under reduced pressure. Specifically, it is essentially made of a polyester resin mixture of a polyester resin mainly based on ethylene glycol and terephthalic acid and a polyester resin mainly based on ethylene glycol and isophthalic acid.

The polyester resin mainly based on ethylene glycol and terephthalic acid in the present invention means a thermoplastic polyester resin which contains terephthalic acid components in an amount of more than 70 mol%, preferably more than, 90 mol % of the whole dicarboxylic acid components, and ethylene glycol components in an amount of more than 70 mol%, preferably more than 90 mol % of the whole glycol components. The other part of the dicarboxylic acid components may be, for instance, aromatic dicarboxylic acid such as isophthalic acid, diphenylether-4,4-dicarboxylic acid and naphthalene-1,4 (or 2,6)-dicarboxylic acid; aliphatic dicarboxylic acid such as oxalic acid, succinic acid, adipic acid, sebacic acid and undeca-dicarboxylic acid; and hexahydroterephthalic acid. The other part of the glycol components may be, for instance, aliphatic glycol such as propylene glycol, 1,4-butandiol and neopentyl glycol; cyclohexane dimethanol; and aromatic dihydroxy compounds such as bisphenol. So far as the amount of the terephthalic acid components and ethylene glycol components are within the above ranges, respectively, the resin may consist of a copolymer thereof or a mixture of polyethylene terephthalate (PET) and other polyester.

The molecular weight of the polyester resin mainly based on ethylene glycol and terephthalic acid according to the invention is not critical, though it should be within the range capable of forming the tubular member, of course. It may be specified by using its intrinsic viscosity ( η) at 25 °C orthochlorophenol, which is generally more than 0.6 dl/g, preferably within the range of 0.8 to 0.85 dl/g.

The polyester resin mainly based on ethylene glycol and isophthalic acid in the present invention means a polyester copolymer which contains isophthalic acid components in an amount of 50 to 100 mol% of the whole dicarboxylic acid components; terephthalic acid components in an amount up to 50 mol% of the whole dicarboxylic acid components; ethylene glycol components in an amount of 10 to 95 mol%, preferably 15 to 90 mol %, more preferably 50 to 90 mol% of the whole dihydroxy compound components; and 1,3-bis (2-hydroxyethoxy)benzene or 1,4-bis(hydroxyethoxy)benzene components in an amount of 5 to 90 mol %, preferably 10 to 85 mol %, more preferably 10 to 50 mol% of the whole dihydroxy compound components. If the amount of the isophthalic acid components is below 20 mol %, sufficient gas barrier properties of the tubular member cannot be obtained. If the amount of the 1,3-bis(2-hydroxyethoxy)benzene or 1,4-bis(hydroxyethoxy)benzene components is below 5 mol%, it is hard to restrain the generation of undesirable oligomers. If the amount of the 1,3-bis(2-hydroxyethoxy)benzene or 1,4-bis(hydroxyethoxy)benzene components is above 90 mol %, the rate of the polycondensation of the resin considerably decreases.

Also the molecular weight of the polyester resin mainly based on ethylene glycol and isophthalic acid according to the invention is not critical, though it should be also within the range capable of forming the tubular member. It may also be specified by using its intrinsic viscosity ( η) at 25 °C orthochlorophenol, which is also more than 0.6 dl/g, preferably within the range of 0.8 to 0.85 dl/g.

The polyester resin mixture of which the tubular member is essentially made consists of the above-mentioned polyester resin mainly based on ethylene glycol and terephthalic acid in an amount of 5 to 95% in weight, preferably 50 to 90 % in weight and the above-mentioned polyester resin mainly based on ethylene glycol and isophthalic acid in an amount of 95 to 5% in weight, preferably 50 to 10 % in weight. It is preferable that the amount of the polyester resin mainly based on ethylene glycol and isophthalic acid is more than 20% in weight because superior gas barrier properties can be obtained. It is also preferable that the amount of the polyester resin mainly based on ethylene glycol and isophthalic acid is less than 50 % in weight because the heat and shock resistances of the tubular member scarcely decreases. When the ratio of the polyester resin mainly based on ethylene glycol and isophthalic acid to the polyester resin mainly based on ethylene glycol and terephthalic acid is 30 % in weight in the case that the polyester resin mainly based on ethylene glycol and isophthalic acid is mixed with pure polyethylene terephthalate (PET), double the gas barrier properties of the PET can be obtained. If the ratio of the polyester resin mainly based on ethylene glycol and isophthalic acid is too little, the aimed improvement cannot be attained. If the ratio is too much, affections of the polyester resin mainly based on ethylene glycol and isophthalic acid to the final product in brittleness and color become considerable. The ratio within the range of 10 to 50% in weight of the polyester resin mainly based on ethylene glycol and isophthalic acid to the polyester resin mainly based on ethylene glycol and terephthalic acid is preferable in view of the gas barrier and other physical properties. More preferable range thereof is 20 to 35 % in weight.

The above polyester resin mixture may be prepared by the manner that the polyester resin mainly based on ethylene glycol and terephthalic acid and the polyester resin mainly based on ethylene glycol and isophthalic acid are mixed with each other within the above-mentioned range by various known methods, for instance, using a Henschel mixer, a V-blender, a ribbon blender, a tumbler or the like. The resulting mixture may be kneaded with a single or twin screw extruder, a kneader, a Banbury mixer or the like. Granulation or mill techniques may also be used.

To the above polyester resin mixture, various additive agents generally used for polyester resin such as heat stabilizers, stabilizers for weather resistance, antistatic agents, lubricants, mold release agents, dispersants, pigments and dyes may be added within the scope of the present invention.

The tubular member 2 may be made of the above polyester resin mixture by injection molding, biaxial orientation, vacuum forming, compression molding or the like.

In the case of tubes for use in coagulating blood or counting red or white blood cells, it is preferable to treat the inner surface of the tubular member 2 to be hydrophilic so as to prevent blood cells from adhering to the inner surface. This treatment can be carried out by coating the inner surface of the tubular member 2 with hydrophilic materials such as water-soluble silicone resin, polyvinyl alcohol and polyvinyl pyrrolidone. An anticoagulant agent such as heparin powder and EDTA-2K may be applied to the inner surface of the tubular member 2 or contained in the tubular member 2. To the contrary, blood-coagulation promoter may be applied to the inner surface of the tubular member 2 or contained in the tubular member 2.

As shown in Fig. 1, a coagulation promoter member 20 consisting of a film, a filter paper, a non-woven fabric or the like to which blood-coagulation promoter has been applied or into which blood-coagulation promoter has been permeated, may be enclosed in the tubular member 2. Instances of the blood-coagulation promoter are silica sands having the particle diameters of 0.4 to 20 µm, crystal silica having the particle diameters less than 5 µm and the average particle diameter of 1.1 µm (for instance, Min-U-Sil, the trade name of Pennsylvania Glass Sand Company), diatomite, fine glass particles, kaolin, bentonite, protamine sulfate and thrombin.

A serum separator may be contained in the tubular member 2. The serum separator is a thixotropic gel material having the specific gravity intermediate between those of serum and blood cell components to be examined. For instance, a material containing as the principal ingredients α-olefin-maleic diester copolymer to which modifiers for viscosity and specific gravity have been added, is usable for this purpose.

In the embodiment shown in Fig. 1, the closure member 3 comprises a gas-barrier member 4 having an adhesive film 6 disposed on the lower surface thereof and a sealing member 5 mounted on the upper surface of the gas-barrier member 4.

The gas-barrier member 4 is for hermetically closing the open end of the tubular member 2 to keep the interior of the tubular member 2 under reduced pressure. The gas-barrier member 4 comprises a gas-barrier film (7) made of a material having high gas-barrier properties, for instance, a metal foil such as an aluminum foil or a resin such as ethylene-vinyl alcohol copolymer and polyvinylidene chloride. The adhesive film 6 is disposed on the lower surface of the gas-barrier member 4 for mounting the closure member 3 to the open end of the tubular member 2. The adhesive film 6 is made of a resin possible to be welded to the polyester resin of the tubular member 2 and having the ability of easy-peeling. The adhesive film 6 is preferably made of a modified polyester resin, which has the lower softening point than the polyester resin of the tubular member 2. The modified polyester resin should have good adhesion to polyethylene terephthalate and the moderate softening and glass transition points. It may consist of aromatic dicarboxylic acid such as terephthalic acid and isophthalic acid, and diol such as ethylene glycol, 1,4-butanediol, diethylene glycol and neopentyl glycol. The modified polyester resin preferably has the softening point within the range of 80 to 170°C (measured by the ring and ball method according to K2531 of the Japanese Industrial Standards) and the glass transition point within the range of -30 to 80°C (measured by DSC method).

In the embodiment shown in Fig. 1, the closure member 3 is provided with a tab 9 for detaching the closure member 3 from the tubular member 2.

Referring next to Fig.2, the gas-barrier member 4 is preferably provided with a resin film 10 disposed on the lower surface of the above-mentioned gas-barrier film, that is, between the gas-barrier film 7 and the adhesive film 6. This resin film 10 is for improving the mechanical strength of the whole film composite and may be made of an oriented PET film. A preferable form of the closure member 3 will be described. The closure member 3 comprises a gas-barrier film 7, a resin film 10 disposed on the lower surface of the gas-barrier film 7, and the adhesive film 6 disposed on the lower surface of the resin film 10. The closure member 3 may be provided with a printing layer 11 disposed on the upper surface of the gas-barrier film 7 for indication of sort, etc. An overcoat 12 such as a cellulose coating layer may be provided to protect the printing layer 11.

The sealing member 5 should be of a material capable of sealing a puncture opening to maintain liquid-tightness both when the hollow needle segment of the tube holder or the like (not shown) is thrusted into and withdrawn from the closure member 3. The sealing member 5 may be made of rubber such as natural rubber, isoprene rubber, chloroprene rubber and silicone rubber, and resin such as thermoplastic elastomer, for instance, styrene-butadiene-styrene (SBS) block copolymer.

The shape of the sealing member 5 is as shown in Fig. 1, which has a plane bottom surface forming the adhesive surface to the gas-barrier member 4, and a recessed blood-receiving portion 13 formed at the upper center of the sealing member 5.

The blood-receiving portion 13 is for receiving and isolating blood which is adhered to the sealing member 5 when the hollow needle segment of the tube holder or the like ( see page 2 and figure 8 of EP 0 352 322 ) is withdrawn from the closure member 3. The sealing member 5 is disposed substantially at the center of the upper surface of the gas-barrier member 4. The outline of the sealing member 5 may be one of circles and other circular shapes including ellipses, and polygons such as quadrangles and pentagons. Alternatively, the sealing member may cover the whole upper surface of the gas-barrier member 4. Although it is preferable to dispose the sealing member at the upper surface of the gas-barrier member 4, the sealing member may be disposed at the lower surface of the gas-barrier member 4.

The closure member 3 including the adhesive film as its lowermost layer can be attached in gas-tight manner to the flange 8 of the tubular member 2, or onto the fringe of the open end of the tubular member if such a flange is not provided, by welding with heat, ultrasonics or high frequency.

A conventional rubber plug may be used as closure member for the tubular member 2 instead of such a film-type closure member as described above.

Reduced-pressure state in the tubular member 2 can be established by the manner that the closure member 3 is attached to the tubular member 2 under reduced atmospheric-pressure.

An experiment for proving the effect of the invention will be described.

### Example

Tubular members used in the experiment had the shape as shown in Fig.1 and the dimensions that the inner diameter at the open end, the thickness and the tapering rate were 13.4 mm, 1.0 mm and 15/1000, respectively. A flange having the outer diameter of 17.3 mm and the thickness of 2.0 mm was provided at the open end of every tubular member. Tubular members according to the invention were made by injection molding from a polyester resin mixture of polyethylene terephthalate (J025 available by Mitsui PET Corporation) and a polyester resin mainly based on ethylene glycol and isophthalic acid (B010; polyester copolymer consisting of terephthalic acid : isophthalic acid / ethylene glycol : 1,3-bis(2-hydroxy)benzene = 10 : 90 / 85 : 15) where the polyethylene terephthalate resin : the polyester resin mainly based on ethylene glycol and isophthalic acid = 7 : 3. They could be easily formed without whitening at the gate position and stopping-up of the gate of the injection molding machine. Every closure member used in the experiment comprised a gas-barrier member which was made of a film consisting of 12 µm PET (SPET available by Toyobo Co., Ltd.) as the uppermost layer, a 30 µm aluminum film as the intermediate layer, and a 15 µm modified polyester-coated PET film as the lowermost layer. The closure member was provided with a sealing member made of natural rubber and having the diameter of 7.0 mm and the thickness of 2.0 mm. A recess having the diameter of 3.0 mm and the depth of 0.8 mm was formed at the upper center of the sealing member.

A coagulation promoter-coated PET film (10 µm thick) was prepared by dipping a PET film into an ethanol solution in which crystal silca powder having the average particle diameter of 2 µm and polyvinyl pyrrolidone were dispersed. Coagulation promoter members each having the diameter of 11 mm were punched from the coagulation promoter-coated PET film.

Water-soluble silicone was sprayed onto the inner surface of the tubular member so as to prevent blood clot from adhering. After inserting the coagulation promoter member, the tubular member was sealed with the closure member by the manner that the gas-barrier member of the closure member was welded to the tubular member with heat under reduced pressure. The above-mentioned sealing member was sealed on the upper surface of the gas-barrier member with adhesion. The blood-collecting tube thus obtained was regulated in its reduced internal pressure to be able to collect the initial amount of blood of 7.0 ml.

In this experiment, blood-collecting tubes sterilized by exposure to gamma radiations (1.5 Mrad) were also prepared. They, however, showed no difference in experimental results from those not sterilized.

### Comparative Example

For comparative examples, tubular members were made in the similar manner but using only polyethylene terephthalate (J025 available by Mitsui PET Corporation) instead of the above-mentioned polyester resin mixture. Blood-collecting tubes each of which was for collecting the initial amount of blood of 7.0 ml, were prepared using these tubular members in the same manner as those of the above-mentioned examples of the invention.

### Experiment

When the change of the capability of collecting blood has observed at room temperature, the experimental results are shown in the following table 1, where the capability of collecting blood was measured by the manner that each tube was made to suck water, and the the measurement temperature and pressure were compensated.

**Table 1**

| Years | 0.5 | 1 | 1.5 | 2 |
|---|---|---|---|---|
| Example of the invention | 6.7 ml | 6.5 ml | 6.3 ml | 6.1 ml |
| Comparative example | 6.5 ml | 6.1 ml | 5.7 ml | 5.4 ml |
| Initialization: 7.0 ml | | | | |

A blood-collecting tube of the present invention is advantageous because the interior of the tube can be thoroughly observed owing to no whitening upon injection molding as well as because the capability of collecting blood decreases little with time owing to its high gas barrier properties.

## Claims

1. A blood collecting tube comprising a tubular member (2) made of synthetic resin which has an open end and a closed bottom, and a closure member (3) which is for closing said open end of said tubular member (2) and allows a puncture needle to pierce therethrough, the interior of said blood collecting tube being suitable to be kept under reduced pressure,
characterized in that said tubular member (2) is made of a mixture of
a) a polyester resin mainly based on ethylene glycol and terephthalic acid,
wherein terephthalic acid components form more than 70 mol % of the whole dicarboxylic acid components and ethylene glycol components form more than 70 mol % of the whole glycol components present,
and
b) a polyester resin mainly based on ethylene glycol and isophthalic acid,
wherein 50 to 100 mol % of the whole dicarboxylic acid components are isophthalic acid components, and up to 50 mol % of said dicarboxylic acid components are terephthalic acid components, and
ethylene glycol components form 10 to 95 mol % of the whole dihydroxy compound components, with at least one of 1,3-bis(2-hydroxyethoxy)benzene and 1,4-bis (hydroxyethoxy)benzene forming 5 to 90 mol% of the whole dihydroxy compound components.

2. A blood collecting tube according to claim 1, characterized in that said polyester resin mainly based on ethylene glycol and terephthalic acid contains terephthalic acid components in an amount of more than 90 % of the whole dicarboxylic acid components thereof.

3. A blood collecting tube according to claim 1, characterized in that said polyester resin mainly based on ethylene glycol and terephthalic acid contains ethylene glycol components in an amount of more than 90 mol % of the whole glycol components thereof.

4. A blood collecting tube according to claim 1, characterized in that said polyester resin mainly based on ethylene glycol and isophthalic acid contains ethylene glycol components in an amount of 15 to 90 mol % of the whole dihydroxy compound components with at least one of 1,3-bis(2-hydroxyethoxy)benzene and 1,4-bis(hydroxyethoxy)benzene components in an amount of 10 to 85 mol % of the whole dihydroxy compound components.

5. A blood collecting tube according to claim 4, characterized in that said polyester resin mainly based on ethylene glycol and isophthalic acid contains ethylene glycol components in an amount of 50 to 90 mol % of the whole dihydroxy components with at least one of 1,3-bis(2-hydroxyethoxy)benzene and 1,4-bis(hydroxyethoxy)benzene components in an amount of 10 to 50 mol % of the whole dihydroxy compound components.

6. A blood collecting tube according to claim 1, characterized in that said mixture consists of
a) 5 to 95 % weight of said polyester resin mainly based on ethylene glycol and terephthalic acid, and
b) 95 to 5 % by weight of said polyester resin mainly based on ethylene glycol and isophthalic acid.

7. A blood collecting tube acording to claim 6, characterized in that said mixture consists of
a) 50 to 90 % by weight of said polyester resin mainly based on ethylene glycol and terephthalic acid, and
b) 50 to 10 % by weight of said polyester resin mainly based on ethylene glycol and isophthalic acid.

8. A blood collecting tube according to claim 7, characterized in that said mixture consists of 50 to 20 % by weight of polyester resin mainly based on ethylene glycol and isophtalic acid.

9. A blood collecting tube according to claim 1, characterized in that the inner surface of said tubular member (2) is coated with a hydrophilic material.

10. A blood collecting tube according to claim 1, characterized in that an anticoagulant agent is applied to the inner surface of said tubular member (2) or contained in said tubular member (2).

11. A blood collecting tube according to claim 1, characterized in that blood-coagulation promoter is applied to the inner surface of said tubular member (2) or contained in said tubular member (2).

12. A blood collecting tube acccording to claim 1, characterized in that said tubular member (2) has a flange (8) at said open end thereof.

13. A blood collecting tube according to claim 1, characterized in that said closure member (3) comprises a gas-barrier member (4) having an adhesive film (6) disposed on a lower surface thereof, and a sealing member (5) disposed on an upper surface of said gas-barrier member (4).

14. A blood collecting tube according to claim 13, characterized in that said gas-barrier member (4) comprises a gas-barrier film (7).

15. A blood collecting tube according to claim 13, characterized in that said closure member (3) has a tab (9) for detaching said closure member (3) from said tubular member (2).

16. A blood collecting tube according to claim 13, characterized in that said sealing member (5) has a recessed blood-receiving portion (13) at the upper surface thereof.

17. A blood collecting tube according to claim 1, characterized in that said closure member (3) is welded to said tubular member (2).

## Patentansprüche

1. Blutsammelröhrchen, umfassend ein ein offenes Ende und einen geschlossenen Boden aufweisendes Rohrteil (2) aus einem Kunstharz und ein Verschlußteil (3) zum Verschließen des offenen Endes des Rohrteils (2), das durch eine Durchstechnadel durchbohrbar ist, wobei sich das Innere des Blutsammelröhrchens unter vermindertem Druck halten läßt, dadurch gekennzeichnet, daß das Rohrteil (2) aus einem Gemisch aus
a) einem hauptsächlich auf Ethylenglykol und Terephthalsäure basierenden Polyesterharz, bei dem die Terephthalsäurekomponenten mehr als 70 Mol% der gesamten Dicarbonsäurekomponenten und die Ethylenglykolkomponenten mehr als 70 Mol% der gesamten vorhandenen Glykolkomponenten ausmachen, und
b) einem hauptsächlich auf Ethylenglykol und Isophthalsäure basierenden Polyesterharz, bei dem 50 bis 100 Mol% der gesamten Dicarbonsäurekomponenten aus Isophthalsäurekomponenten und bis zu 50 Mol% der Dicarbonsäurekomponenten aus Terephthalsäurekomponenten bestehen und die Ethylenglykolkomponenten 10 bis 95 Mol% der gesamten Dihydroxyverbindungskomponenten ausmachen, wobei mindestens eines von 1,3-Bis(2-hydroxyethoxy)benzol und 1,4- Bis(hydroxyethoxy)benzol 5 bis 90 Mol% der gesamten Dihydroxyverbindungskomponenten bildet,
hergestellt ist.

2. Blutsammelröhrchen nach Anspruch 1, dadurch gekennzeichnet, daß das häuptsächlich auf Ethylenglykol und Terephthalsäure basierende Polyesterharz mehr als 90 % seiner gesamten Dicarbonsäurekomponenten an Terephthalsäurekomponenten enthält.

3. Blutsammelröhrchen nach Anspruch 1, dadurch gekennzeichnet, daß das hauptsächlich auf Ethylenglykol und Terephthalsäure basierende Polyesterharz mehr als 90 Mol% seiner gesamten Glykolkomponenten an Ethylenglykolkomponenten enthält.

4. Blutsammelröhrchen nach Anspruch 1, dadurch gekennzeichnet, daß das hauptsächlich auf Ethylenglykol und Isophthalsäure basierende Polyesterharz 15 bis 90 Mol% der gesamten Dihydroxyverbindungskomponenten an Ethylenglykolkomponenten enthält, wobei mindestens eine der 1,3-Bis(2-hydroxyethoxy)benzol- und 1,4- Bis(hydroxyethoxy)benzolkomponenten 10 bis 85 Mol% der gesamten Dihydroxyverbindungskomponenten ausmacht.

5. Blutsammelröhrchen nach Anspruch 4, dadurch gekennzeichnet, daß das hauptsächlich auf Ethylenglykol und Isophthalsäure basierende Polyesterharz 50 bis 90 Mol% der gesamten Dihydroxykomponenten an Ethylenglykolkomponenten enthält, wobei mindestens eine der 1,3-Bis(2-hydroxyethoxy)benzol- und 1,4-Bis(hydroxyethoxy)benzolkomponenten 10 bis 50 Mol% der gesamten Dihydroxyverbindungskomponenten ausmacht.

6. Blutsammelröhrchen nach Anspruch 1, dadurch gekennzeichnet, daß das Gemisch aus
a) 5 bis 95 Gew.% des hauptsächlich auf Ethylenglykol und Terephthalsäure basierenden Polyesterharzes und
b) 95 bis 5 Gew.% des hauptsächlich auf Ethylenglykol und Isophthalsäure basierenden Polyesterharzes
besteht.

7. Blutsammelröhrchen nach Anpruch 6, dadurch gekennzeichnet, daß das Gemisch aus
a) 50 bis 90 Gew.% des hauptsächlich auf Ethylenglykol und Terephthalsäure basierenden Polyesterharzes und
b) 50 bis 10 Gew.% des hauptsächlich auf Ethylenglykol und Isophthalsäure basierenden Polyesterharzes
besteht.

8. Blutsammelröhrchen nach Anpruch 7, dadurch gekennzeichnet, daß das Gemisch aus 50 bis 20 Gew.% des hauptsächlich auf Ethylenglykol und Isophthalsäure basierenden Polyesterharzes besteht.

9. Blutsammelröhrchen nach Anspruch 1, dadurch gekennzeichnet, daß die Innenwand des Rohrteils (2) mit einem hydrophilen Material beschichtet ist.

10. Blutsammelröhrchen nach Anspruch 1, dadurch gekennzeichnet, daß ein Antikoagulationsmittel auf die Innenwand des Rohrteils (2) appliziert oder im Rohrteil (2) enthalten ist.

11. Blutsammelröhrchen nach Anspruch 1, dadurch gekennzeichnet, daß ein die Blutkoagulation förderndes Mittel auf die Innenwand des Rohrteils (2) appliziert oder im Rohrteil (2) enthalten ist.

12. Blutsammelröhrchen nach Anspruch 1, dadurch gekennzeichnet, daß das Rohrteil (2) an seinem offenen Ende einen vorstehenden Rand (8) aufweist.

13. Blutsammelröhrchen nach Anspruch 1, dadurch gekennzeichnet, daß das Verschlußteil (3) ein Gasabsperrteil (4) mit einem an seiner Unterseite vorgesehenen Klebefilm (6) und ein an der Oberseite des Gasabsperr-teils (4) vorgesehenes Versiegelungsteil (5) aufweist.

14. Blutsammelröhrchen nach Anspruch 13, dadurch gekennzeichnet, daß das Gasabsperrrteil (4) einen Gassperre-film (7) umfaßt.

15. Blutsammelröhrchen nach Anspruch 13, dadurch gekennzeichnet, daß das Verschlußteil (3) einen Streifen (9) zum Lösen des Verschlußteils (3) von dem Rohrteil (2) aufweist.

16. Blutsammelröhrchen nach Anspruch 13, dadurch gekennzeichnet, daß das Versiegelungsteil (5) auf seiner Oberseite einen eingebuchteten Blutaufnahmebereich (13) aufweist.

17. Blutsammelröhrchen nach Anspruch 1, dadurch gekennzeichnet, daß das Verschlußteil (3) an das Rohrteil (2) angeschweißt ist.

## Revendications

1. Tube de prélèvement de sang, comprenant un élément tubulaire (2) en résine synthétique et qui présente une extrémité ouverte et un fond fermé, et un élément de fermeture (3) qui est destiné à fermer ladite extrémité ouverte dudit élément tubulaire (2) et qui peut être transpercé par une aiguille creuse, l'intérieur dudit tube de prélèvement de sang étant apte à être maintenu sous pression réduite, caractérisé en ce que ledit élément tubulaire (2) est fait d'un mélange de
a) une résine de polyester principalement constituée d'éthylèneglycol et d'acide téréphtalique,
dans laquelle le composant acide téréphtalique constitue plus de 70 % en moles de la totalité des composants acides dicarboxyliques et le composant éthylèneglycol constitue plus de 70 % en modes de la totalité des composants glycols présents,
et de
b) une résine de polyester principalement constituée d'éthylèneglycol et d'acide isophtalique,
dans laquelle de 50 à 100 % en moles de la totalité des composants acides dicarboxyliques sont constitués par le composant acide isophtalique et jusqu'à 50 % en moles desdits composants acides dicarboxyliques sont constitués par le composant acide téréphtalique, et le composant éthylèneglycol constitue de 10 à 95 % en modes de la totalité des composants diols, au moins l'un du 1,3-bis(2-hydroxyéthoxy)benzène et du 1,4-bis(2-hydroxyéthoxy)benzène constituant de 5 à 90 % en modes de la totalité des composants diols.

2. Tube de prélèvement de sang, conforme à la revendication 1, caractérisé en ce que ladite résine de polyester principalement constituée d'éthylèneglycol et d'acide téréphtalique contient du composant acide téréphtalique en une quantité représentant plus de 90 % en modes de la totalité de ses composants acides dicarboxyliques.

3. Tube de prélèvement de sang, conforme à la revendication 1, caractérisé en ce que ladite résine de polyester principalement constituée d'éthylèneglycol et d'acide téréphtalique contient du composant éthylèneglycol en une quantité représentant plus de 90 % en moles de la totalité de ses composants glycols.

4. Tube de prélèvement de sang, conforme à la revendication 1, caractérisé en ce que ladite résine de polyester principalement constituée d'éthylèneglycol et d'acide isophtalique contient du composant éthylèneglycol en une quantité représentant de 15 à 90 % en moles de la totalité des composants diols, au moins l'un des composants 1,3-bis(2-hydroxyéthoxy)benzène et 1,4-bis(2-hydroxyéthoxy)benzène se trouvant en une quantité représentant de 10 à 85 % en moles de la totalité des composants diols.

5. Tube de prélèvement de sang, conforme à la revendication 4, caractérisé en ce que ladite résine de polyester principalement constituée d'éthylèneglycol et d'acide isophtalique contient du composant éthylèneglycol en une quantité représentant de 50 à 90 % en moles de la totalité des composants diols, au moins l'un des composants 1,3-bis(2-hydroxyéthoxy)benzène et 1,4-bis(2-hydroxyéthoxy)benzène se trouvant en une quantité représentant de 10 à 50 % en moles de la totalité des composants diols.

6. Tube de prélèvement de sang, conforme à la revendication 1, caractérisé en ce que ledit mélange est constitué de :
a) 5 à 95 % en poids de ladite résine de polyester principalement constituée d'éthylèneglycol et d'acide téréphtalique, et
b) 95 à 5 % en poids de ladite résine de polyester principalement constituée d'éthylèneglycol et d'acide isophtalique.

7. Tube de prélèvement de sang, conforme à la revendication 6, caractérisé en ce que ledit mélange est constitué de :
a) 50 à 90 % en poids de ladite résine de polyester principalement constituée d'éthylèneglycol et d'acide téréphtalique, et
b) 50 à 10 % en poids de ladite résine de polyester principalement constituée d'éthylèneglycol et d'acide isophtalique.

8. Tube de prélèvement de sang, conforme à la revendication 7, caractérisé en ce que ledit mélange est constitué, pour 50 à 20 % en poids, d'une résine de polyester principalement constituée d'éthylèneglycol et d'acide isophtalique.

9. Tube de prélèvement de sang, conforme à la revendication 1, caractérisé en ce que la surface interne dudit élément tubulaire (2) est revêtue d'un matériau hydrophile.

10. Tube de prélèvement de sang, conforme à la revendication 1, caractérisé en ce qu'un agent anticoagulant est étalé sur la surface interne dudit élément tubulaire (2) ou contenu dans ledit élément tubulaire (2).

11. Tube de prélèvement de sang, conforme à la revendication 1, caractérisé en ce qu'un agent favorisant la coagulation du sang est étalé sur la surface interne dudit élément tubulaire (2) ou contenu dans ledit élément tubulaire (2).

12. Tube de prélèvement de sang, conforme à la revendication 1, caractérisé en ce que ledit élément tubulaire (2) présente, à son extrémité ouverte, un rebord (8).

13. Tube de prélèvement de sang, conforme à la revendication 1, caractérisé en ce que ledit élément de fermeture (3) comporte un élément (4) formant barrière pour les gaz et portant un film adhésif (6) disposé sur sa face inférieure, et un élément d'étanchéité (5) disposé sur la face supérieure dudit élément (4) formant barrière aux gaz.

14. Tube de prélèvement de sang, conforme à la revendication 13, caractérisé en ce que ledit élément (4) formant barrière aux gaz comporte un film (7) formant barrière aux gaz.

15. Tube de prélèvement de sang, conforme à la revendication 13, caractérisé en ce que ledit élément de fermeture (3) comporte une languette (9) permettant d'enlever ledit élément de fermeture (3) dudit élément tubulaire (2).

16. Tube de prélèvement de sang, conforme à la revendication 13, caractérisé en ce que ledit élément d'étanchéité (5) présente, à sa surface supérieure, une partie en retrait (13) de rétention de sang.

17. Tube de prélèvement de sang, conforme à la revendication 1, caractérisé en ce que ledit élément de fermeture (3) est soudé audit élément tubulaire (2).
